# EUROPEAN PATENT APPLICATION

(11) **EP 4 491 612 A1**
(43) Date of publication of application: **15.01.2025**
(21) Application number: 23766793.6
(22) Date of filing: 06.03.2023
(51) Int. Cl.: C07C 231/12, C07B 59/00, C25B 3/11, C25B 3/27, C25B 15/023, C25B 15/029, C25B 15/08, A61K 51/00

(54) **METHOD FOR PRODUCING RADIOLABELED ARYL COMPOUND BY ELECTROLYTIC OXIDATION REACTION**

(30) Priority: 08.03.2022 JP 2022035169
(71) Applicant: OSAKA UNIVERSITY, Suita-shi Osaka 565-0871 (JP)
(72) Inventor: FUKASE Koichi, Suita-shi, Osaka 565-0871 (JP); TOYOSHIMA Atsushi, Suita-shi, Osaka 565-0871 (JP); SHINOHARA Atsushi, Suita-shi, Osaka 565-0871 (JP); SHIRAKAMI Yoshifumi, Suita-shi, Osaka 565-0871 (JP); KANEDA Kazuko, Suita-shi, Osaka 565-0871 (JP); SHIMOYAMA Atsushi, Suita-shi, Osaka 565-0871 (JP); KADONAGA Yuichiro, Suita-shi, Osaka 565-0871 (JP)
(74) Representative: Symbiosis IP Limited
(86) International application number: PCT/JP2023/008345
(87) International publication number: WO 2023/171617

(57) **Abstract**

Provided is a novel method for labeling a radionuclide such as ²¹¹At with a high radiochemical yield under more convenient and stable conditions.

A production method of an aryl compound labeled with a radionuclide includes a step of applying a voltage to a solution A containing a radionuclide and electrolytically oxidizing the radionuclide; and a step of mixing the solution A and a solution B containing a compound S having a halogenated aryl group to substitute a halogen atom on the aryl group of the compound S with the radionuclide.

## Description

### Technical Field

The present invention relates to a production method of an aryl compound labeled with a radionuclide and a flow electrolysis apparatus suitable for the production method.

### Background Art

The α-ray nuclear medicine therapy is a therapy in which a drug labeled with an α-ray emitting nuclide is administered to a cancer patient, the drug is accumulated in a cancer tissue, and the cancer is irradiated with an α-ray from the body to kill the cancer. The α-ray has high energy and high ability of killing cancer cells, and on the other hand, since a range is as short as several human cells, the α-ray does not invade surrounding organs and normal cells. Therefore, when the α-ray emitting nuclide can be selectively accumulated in cancer cells, only the cancer cells can be killed, and thus it becomes a new therapy for cancer in which high efficacy and safety are expected. For example, Xofigo [radiopharmaceutical-based radium chloride (²²³Ra) injection solution] is approved as a therapeutic agent for castration-resistant prostate cancer (CRPC) having bone metastasis as an α-ray nuclear medicine therapeutic agent, and further, a drug in which ²²⁵Ac is bound to a molecular target drug targeting a tumor has shown an extremely remarkable anticancer effect in clinical trials, and thus has attracted great attention.

On the other hand, development of an anticancer therapeutic agent using astatin-211 (²¹¹At), which is one of α-ray emitting nuclides, has been conducted. The pharmacokinetics of ²¹¹At in a patient body can be analyzed by SPECT imaging. From the viewpoint of drug production, since ²¹¹At is halogen, ²¹¹At can be directly bonded to carbon atoms or the like of a lot of organic compounds. Therefore, ²¹¹At has an advantage that it can be introduced from various low molecular weight drugs to high molecular weight drugs.

As a method for introducing ²¹¹At into an organic compound and labeling the compound, for example, a method in which a boronophenyl group-containing compound is used as a starting material and a boronic acid group is substituted with ²¹¹At in the presence of KI to provide a ²¹¹At labeled compound (borono method) is known (Patent Literature 1). This method has been developed by the applicant of the present application, and is characterized in that a compound having a boronophenyl group can be labeled with ²¹¹At and has a wide application range. On the other hand, there is a restriction that the starting material is limited to those having a boronic acid group.

In addition, as a method other than the borono method, the applicant has developed a ²¹¹At labeling method (mercury method) for α-methyl-L-tyrosine (AMT), which is a cancer accumulation molecule, by applying a known mercury method (CancerSci. 2021, 112, 1132). This method exhibits an excellent labeling rate, but has a problem that it is not a method suitable for pharmaceutical development.

### Citation List

### Patent Literature

Patent Literature 1: WO 2019/027059 A

### Non Patent Literature

Non Patent Literature 1: Kaneda-Nakasima et al. CancerSci. 2021, 112, 1132-1140.

### Summary of Invention

### Technical Problem

From such a background, an object of the present invention is to provide a novel method for labeling a radionuclide such as ²¹¹At with a high radiochemical yield under more convenient and stable conditions.

### Solution to Problem

As a result of intensive studies to solve the above problems, the present inventors have found that by using an electrolytic oxidation reaction, a halogen substituent on an aryl group can be specifically substituted with a radionuclide such as ²¹¹At by transferring electrons through an electrode without using a highly toxic reactant such as an oxidizing agent, and have completed the present invention. In particular, when a compound in which the halogen on the aryl group is iodine is used as a starting material, since the chemical stability of the starting material and the synthesis intermediate is higher than that of the borono method, it can be said that the method can be applied to a wider range of compounds.

That is, in one aspect, the present invention relates to a production method of an aryl compound labeled with a radionuclide such as ²¹¹At, and more specifically, provides:
<1> A production method of an aryl compound labeled with a radionuclide, the method including: a step of applying a voltage to a solution A containing a radionuclide and electrolytically oxidizing the radionuclide; and a step of mixing the solution A and a solution B containing a compound S having a halogenated aryl group to substitute a halogen atom on the aryl group of the compound S with the radionuclide;
<2> The production method according to <1>, in which each of the steps is performed while allowing the solution A and/or the solution B to flow in a flow path at an arbitrary flow rate;
<3> The production method according to <1>, in which each of the steps is performed without allowing the solution A and/or the solution B to flow;
<4> The production method according to <2> or <3>, in which the application of the voltage is performed in an electrolytic bath installed in a flow path;
<5> The production method according to any one of <1> to <4>, in which a radiochemical purity of the aryl compound labeled with the radionuclide is 80.9% or more;
<6> The production method according to any one of <1> to <4>, in which a radiochemical yield of the aryl compound labeled with the radionuclide is 56.3% or more;
<7> The production method according to any one of <1> to <4>, in which a radiochemical purity and a radiochemical yield of the aryl compound labeled with the radionuclide are 71.5% or more and 55.9% or more, respectively;
<8> The production method according to any one of <1> to <7>, in which the voltage is 800 mV or more (vs Ag/AgCl) ;
<9> The production method according to any one of <1> to <7>, in which the voltage is 800 to 1,200 mV (vs Ag/AgCl) ;
<10> The production method according to any one of <1> to <7>, in which the voltage is 800 to 1,000 mV (vs Ag/AgCl) ;
<11> The production method according to any one of <1> to <10>, in which the solution A contains at least one anion selected from the group consisting of an iodide ion, a bromide ion, a chloride ion, and a fluoride ion;
<12> The production method according to <11>, in which a concentration of the anion is in a range of 0.01 M to 0.3 M;
<13> The production method according to any one of <1> to <12>, in which the radionuclide is selected from the group consisting of ²¹¹At, ²¹⁰At, ¹²³I, ¹²⁴I, ¹²⁵I, ¹³¹I, ⁷⁶Br, and ¹⁸F;
<14> The production method according to any one of <1> to <13>, in which the radionuclide is ²¹¹At, and the halogen is an iodine atom;
<15> The production method according to any one of <1> to <14>, in which the aryl group in the compound S is a C₆₋₁₄ aryl group optionally having a substituent;
<16> The production method according to any one of <1> to <15>, in which the compound S is an amino acid derivative, a peptide, or a protein;
<17> The production method according to any one of <1> to <16>, in which the compound S is a compound represented by the following Formula (I) or a salt thereof: 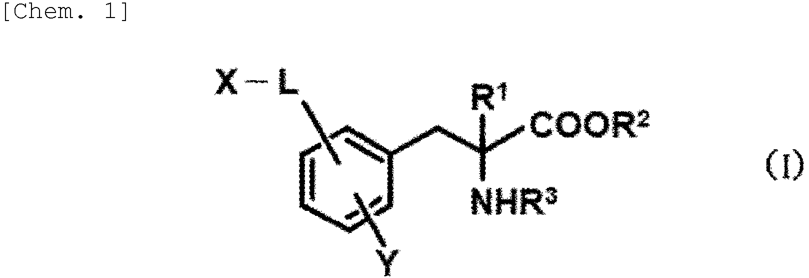 (in the formula, X is a halogen atom; Y is a hydrogen atom or 1 to 4 arbitrary substituents; L is a direct bond or an optionally substituted C₁-C₅ alkylene group; R¹ is a hydrogen atom or an optionally substituted C₁-C₅ alkyl group; R² is a hydrogen atom, a C₁-C₅ alkyl group, a C₆-C₁₄ aryl group, or a C₇-C₁₆ arylalkyl group; and R³ is a hydrogen atom, a C₁-C₃ alkyl group, or an acyl group);
<18> The production method according to <17>, in which at least one Y is a hydroxyl group and R³ is an acyl group; and
<19> The production method according to <18>, in which the protein is an antibody or an antibody fragment.
   In another aspect, the present invention relates to an electrolysis apparatus suitable for the production method, and more specifically, provides:
<20> A flow electrolysis apparatus for use in the production method according to any one of <1> to <19>, the flow electrolysis apparatus including: a plurality of inlets for injecting a solution; a plurality of flow paths through which the solution flows, the plurality of flow paths extending from the inlets; a mixing point at which the plurality of flow paths merge; a discharge flow path extending downstream from the mixing point; an outlet connected to the discharge flow path; an electrolytic bath provided between any one inlet of the plurality of inlets and the mixing point; a voltage application device connected to the electrolytic bath; and a liquid feeding pump for feeding the solution at a desired flow rate;
<21> The flow electrolysis apparatus according to <20>, further including a sample injection portion for adding a solution containing a radionuclide between the inlet and the electrolytic bath;
<22> A stopped-flow type electrolysis apparatus for use in the production method according to any one of <1> to <19>; and
<23> The stopped-flow type electrolysis apparatus according to <22>, further including a sample injection portion for adding a solution containing a radionuclide.

### Advantageous Effects of Invention

According to the present invention, a wide range of compounds having a halogenated aryl group can be radiolabeled with an excellent radiochemical yield under stable conditions without using a highly toxic reactant such as an oxidizing agent.

In addition, the method of the present invention is not limited to the case of performing an electrolytic oxidation reaction in a solution stored in a bulk reaction vessel as in the conventional electrolytic oxidation reaction, and a flow electrolysis apparatus is used, such that it is possible to perform an electrolytic oxidation reaction while allowing a reaction solution to flow, and it is also possible to automate a reaction step. The use of the flow electrolysis apparatus has an advantage that a surface area of an electrode is easily increased, but there is no problem even when the flow electrolysis apparatus is not used (for example, a stopped-flow type electrolysis apparatus) as long as the surface area of the electrode required for the electrolytic oxidation step is secured.

### Brief Description of Drawings

Fig. 1 is a potential-pH diagram of astatin electrolysis and a diagram illustrating positions of an applied voltage and pH in the present study by a dashed circle frame.
Fig. 2 is a schematic view illustrating a configuration of a flow electrolysis apparatus used in Example 1.
Fig. 3 is a flowchart illustrating a first experimental protocol used in Example 1.
Fig. 4 is a graph showing a relationship between a radiochemical purity of a labeled compound obtained and an applied voltage.
Fig. 5 is a graph showing a relationship between a radiochemical yield of a labeled compound obtained and an applied voltage.
Fig. 6 is an HPLC diagram comparing a labeled compound obtained with a preparation.
Fig. 7 is a graph showing a relationship between a residual ratio of N-acetyl-3-iodo-L-tyrosine and a concentration of NaI.

### Mode for Carrying Out the Invention

Hereinafter, embodiments of the present invention will be described. The scope of the present invention is not limited to these descriptions, and examples other than those shown below can be appropriately modified and implemented without impairing the gist of the present invention.

### 1. Definition

In the present specification, examples of "halogen" or "halogen atom" include a fluorine atom, a chlorine atom, a bromine atom, and an iodine atom.

In the present specification, the "alkyl or alkyl group" may be any of linear alkyl, branched alkyl, cyclic alkyl, or an aliphatic hydrocarbon group composed of a combination thereof. The number of carbon atoms in the alkyl group is not particularly limited, and is, for example, 1 to 20 carbon atoms (C₁₋₂₀), 1 to 15 carbon atoms (C₁₋₁₅), or 1 to 10 carbon atoms (C₁₋₁₀). In the present specification, the alkyl group may have one or more arbitrary substituents. For example, C₁₋₈ alkyl includes methyl, ethyl, n-propyl, isopropyl, n-butyl, isobutyl, sec-butyl, tert-butyl, n-pentyl, isopentyl, neo-pentyl, n-hexyl, isohexyl, n-heptyl, n-octyl, and the like. Examples of the substituent include an alkoxy group, a halogen atom (may be a fluorine atom, a chlorine atom, a bromine atom, or an iodine atom), an amino group, a mono- or disubstituted amino group, a substituted silyl group, and acyl, but are not limited thereto. In a case where the alkyl group has two or more substituents, these substituents may be the same as or different from each other. The same applies to alkyl moieties of other substituents including alkyl moieties (for example, an alkoxy group, an arylalkyl group, and the like).

In the present specification, the "alkylene" is a divalent group composed of a linear or branched saturated hydrocarbon, and examples thereof include methylene, 1-methylmethylene, 1,1-dimethylmethylene, ethylene, 1-methylethylene, 1-ethylethylene, 1,1-dimethylethylene, 1,2-dimethylethylene, 1,1-diethylethylene, 1,2-diethylethylene, 1-ethyl-2-methylethylene, trimethylene, 1-methyltrimethylene, 2-methyltrimethylene, 1,1-dimethyltrimethylene, 1,2-dimethyltrimethylene, 2,2-dimethyltrimethylene, 1-ethyltrimethylene, 2-ethyltrimethylene, 1,1-diethyltrimethylene, 1,2-diethyltrimethylene, 2,2-diethyltrimethylene, 2-ethyl-2-methyltrimethylene, tetramethylene, 1-methyltetramethylene, 2-methyltetramethylene, 1,1-dimethyltetramethylene, 1,2-dimethyltetramethylene, 2,2-dimethyltetramethylene, and 2,2-di-n-propyltrimethylene.

In the present specification, the "aryl or aryl group" may be either a monocyclic or fused polycyclic aromatic hydrocarbon group, and may contain one or more heteroatoms (for example, an oxygen atom, a nitrogen atom, a sulfur atom, or the like) as ring-constituting atoms. In this case, it may also be referred to as "heteroaryl" or "heteroaromatic". Even in a case where aryl is a single ring or a fused ring, it may be bonded at all possible positions. In the present specification, the aryl group may have one or more arbitrary substituents on its ring. Examples of the substituent include an alkoxy group, a halogen atom, an amino group, a mono- or disubstituted amino group, a substituted silyl group, and acyl, but are not limited thereto. In a case where the aryl group has two or more substituents, these substituents may be the same as or different from each other. The same applies to aryl moieties of other substituents including aryl moieties (for example, an aryloxy group, an arylalkyl group, and the like).

In the present specification, the "arylalkyl" represents an alkyl substituted with the aryl. The arylalkyl may have one or more arbitrary substituents. Examples of the substituent include an alkoxy group, a halogen atom, an amino group, a mono- or disubstituted amino group, a substituted silyl group, and an acyl group, but are not limited thereto. In a case where the acyl group has two or more substituents, these substituents may be the same as or different from each other. Typical examples thereof include arylalkylbenzyl and p-methoxybenzyl.

In the present specification, in a case where a certain functional group is defined as "optionally substituted", the type of substituent, a substitution position, and the number of substituents are not particularly limited, and in a case where a certain functional group has two or more substituents, these substituents may be the same as or different from each other. Examples of the substituent include an alkyl group, an alkoxy group, a hydroxyl group, a carboxyl group, a halogen atom, a sulfo group, an amino group, an alkoxycarbonyl group, and an oxo group, but are not limited thereto. Substituents may be further present in these substituents. Examples thereof include a halogenated alkyl group, but are not limited thereto.

The "amide" used in the present specification includes both RNR'CO- (when R = alkyl, alkylaminocarbonyl-) and RCONR'- (when R = alkyl, alkylcarbonylamino-).

The "ester" used in the present specification includes both ROCO- (when R = alkyl, alkoxycarbonyl-) and RCOO- (when R = alkyl, alkylcarbonyloxy-).

In the present specification, a specific substituent can form a ring structure with another substituent, and in a case where such substituents are bonded to each other, those skilled in the art can understand that a specific substitution, for example, a bond to hydrogen is formed. Therefore, in a case where it is described that specific substituents together form a ring structure, those skilled in the art can understand that the ring structure can be formed by a usual chemical reaction and is easily generated. Such ring structures and a formation process thereof are all within the purview of those skilled in the art. In addition, the heterocyclic structure may have an arbitrary substituent on the ring.

In the present specification, the term "ring structure" means a heterocyclic or carbocyclic ring when formed by a combination of two substituents, and such a ring can be saturated, unsaturated, or aromatic. Therefore, the ring structure includes cycloalkyl, cycloalkenyl, aryl, and heteroaryl as defined above.

### 2. Production Method of the Present Invention

The production method of the present invention is a method for producing an aryl compound labeled with a radionuclide, and includes the following steps:
a step a) of applying a voltage to a solution A containing a radionuclide and electrolytically oxidizing the radionuclide; and
a step of b) mixing the solution A and a solution B containing a compound S having a halogenated aryl group to substitute a halogen atom on the aryl group of the compound S with the radionuclide.

In the step a), the radionuclide is converted into a highly electrophilic chemical species by an electrolytic oxidation reaction, and in the step b), the chemical species is brought into contact with the compound S as a substrate to cause an electrophilic substitution reaction with the halogen atom on the aryl group, thereby performing labeling with the radionuclide. Such an electrophilic substitution reaction causes a substitution reaction selectively with the halogen atom on the aryl group without reacting with other atoms in the compound S such as a hydrogen atom on the aryl group. Therefore, the production method of the present invention provides an advantage that a radionuclide can be labeled at a desired position by halogenating an aryl group in a desired compound by any synthetic method including a known method.

The radionuclide contained in the solution A is not particularly limited as long as it can be subjected to the electrolytic oxidation reaction, and typically, examples thereof include ²¹¹At, ²¹⁰At, ¹²³I, ¹²⁴I, ¹²⁵I, ¹³¹I, ⁷⁶Br, and ¹⁸F. When the labeled compound obtained by the production method of the present invention is used as a drug such as a therapeutic agent or a diagnostic agent, it is preferable to use a nuclide having a short half-life. The radionuclide is preferably ²¹¹At (astatin-211).

These radionuclides can be adjusted using techniques known in the art. For example, ²¹¹At can be obtained from bismuth (Bi) using an accelerator (cyclotron). More specifically, a 211At stock solution obtained by irradiating bismuth with helium particles accelerated to 28 MeV by a cyclotron, producing ²¹¹At by a nuclear reaction of ²⁰⁹Bi(α,2n)²¹¹At, and dissolving the collected 211At in water or an organic solvent can be used. As for ²¹⁰At, a ²¹⁰At stock solution can be prepared by irradiating bismuth with helium particles accelerated to 29 MeV or more by a cyclotron, producing ²¹⁰At by a nuclear reaction of ²⁰⁹Bi(α,3n)²¹⁰At, and then performing the same operation as above.

In addition, ¹²³I is available as an aqueous Na¹²³I solution. As for ¹²⁴I, ¹²⁴I can be produced by irradiating tellurium with proton particles accelerated by a cyclotron by a nuclear reaction of ¹²⁴Te(p,n)¹²⁴I. In addition, ¹²⁵I is available as an aqueous Na¹²⁵I solution. In addition, ¹³¹I is available as an aqueous Na¹³¹I solution.

¹⁸F can be produced, for example, by using a synthesis method using ¹⁸F-F₂ gas (¹⁸F⁺ method) or the like. ⁷⁶Br can be similarly adjusted using a known method.

The solvent constituting the solution A is not particularly limited, and water, an organic solvent, or a mixed solvent of water and an organic solvent can be used. As the organic solvent, amides (for example, dimethylformamide, dimethylacetamide, N-methylacetamide, and formamide), alcohols (for example, methanol and ethanol), ethers (for example, dioxane, diethyl ether, and tetrahydrofuran), nitriles (for example, acetonitrile and butyronitrile), amines (for example, ethylenediamine and pyridine), ketones (for example, methyl ethyl ketone), chloroform, and the like can be used. Preferably, the solvent is water, that is, the solution A is an aqueous solution.

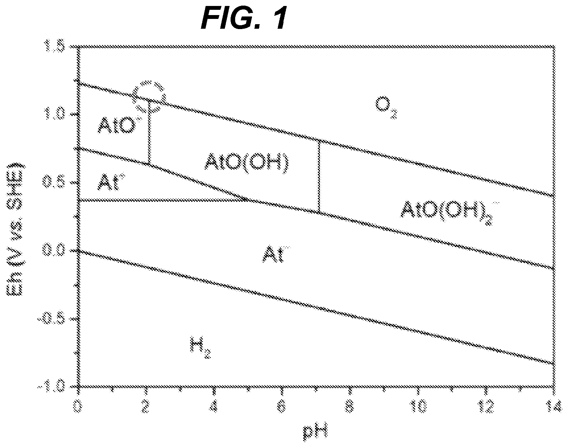

When the solvent contains water, the pH of the solution A can be arbitrarily set. In particular, when the radionuclide is ²¹¹At, the pH is preferably 0 to 5, and more preferably 2 to 4. For example, the pH can be preferably 2.0 ± 2.0, more preferably 2.0 ± 0.5, more preferably 2.0 ± 0.4, more preferably 2.0 ± 0.3, more preferably 2.0 ± 0.2, and still more preferably 2.0 ± 0.1. This is because a salt and an acid act on the chemical species At⁺, AtO⁺, and AtO(OH) predicted to be produced from the potential-pH of At illustrated in Fig. 1 to provide the active species AtOI²⁻, AtOICl-, and AtO(OH)I- estimated by quantum chemical calculation, which is considered to be preferable. However, the reaction can also be performed at any pH other than the above range depending on the type of the radionuclide and the like.

A concentration of the radionuclide in the solution A is not particularly limited, and is preferably 10⁻¹⁶ to 10⁻⁵ M, and more preferably 10⁻¹¹ to 10⁻⁵ M, with respect to 1 Bq to 1,000 GBq of the radionuclide. In particular, when the radionuclide is ²¹¹At, on the other hand, the concentration of the radionuclide is preferably 10⁻⁹ to 10⁻⁵ M because the reaction efficiency is reduced when the concentration of the radionuclide is too low.

In a preferred aspect, it is preferable that the solution A contains at least one anion selected from the group consisting of an iodide ion, a bromide ion, a chloride ion, and a fluoride ion. Due to the presence of these anions, the electrolytic oxidation efficiency and elution rate of the radionuclide can be increased, and AtOI²⁻, AtOICl⁻, and AtO(OH)I⁻, which are active species estimated by quantum chemical calculation, can be obtained. In order to allow these anions to be present, at least one halide salt selected from the group consisting of an iodide salt, a bromide salt, a chloride salt, and a fluoride salt can be added to the solution A. Preferably, these halide salts are alkali metal salts. A concentration of these anions in the solution A can preferably be in a range of 0.01 to 0.3 M, and more preferably in a range of 0.01 to 0.15 M.

As other components, the solution A can contain an electrolyte such as NaCl or an acid such as HCl, HBr, HI, or HF. Due to the presence of the other components, the electrolytic oxidation efficiency and elution rate of the radionuclide can be increased, and AtOI²⁻, AtOICl-, and AtO(OH)I⁻, which are active species estimated by quantum chemical calculation, can be obtained.

Note that, when a flow electrolysis apparatus described below is used, it is also possible to adjust the solution A by adding a solution containing a radionuclide from a sample injection portion to a flow path in the middle of the flow path through which a solution containing only an electrolyte flows.

The application of the voltage in the step a) can be performed by inserting an electrode into an electrolytic bath and applying a voltage between the electrodes from the outside. In a preferred aspect, the electrolytic bath is connected to a flow path through which the solution A flows, and the application of the voltage can be performed in the electrolytic bath installed in the flow path. The electrode may be the same as that used in a general electrochemical reaction, and for example, a carbon electrode, a platinum electrode, a silver electrode, a copper electrode, or the like can be used as the electrode. In addition, it is preferable to use a carbon electrode for an anode and a platinum electrode for a cathode. As the carbon electrode, a porous carbon electrode having a large surface area is preferably used. As the electrolytic bath, a commercially available electrolysis cell or the like can be used.

The electrolysis method may be either a constant current method or a constant voltage method, and a constant voltage method is preferable. In a case of a constant voltage system, it is preferable to use a commercially available potentiostat.

A magnitude of a charge applied can be preferably 800 to 1,000 mV (vs Ag/AgCl), and more preferably 850 to 950 mV (vs Ag/AgCl). However, the magnitude can be appropriately changed according to the type of the radionuclide to be used. A voltage application time (cumulative time) is not particularly limited, and is preferably 1 to 120 minutes, and more preferably 5 to 30 minutes.

The compound S having a halogenated aryl group contained in the solution B used in the step b) is not particularly limited as long as it has a halogenated aryl group as a partial structure. The halogen on the aryl group can be a non-radioisotopic fluorine atom, chlorine atom, bromine atom, or iodine atom, and is preferably an iodine atom. Typically, the radionuclide is ²¹¹At, and the halogen is preferably an iodine atom.

The aryl group in the compound S is preferably a C₆₋₁₄ aryl group optionally having a substituent. Examples of the "C₆₋₁₄ aryl group" include phenyl, 1-naphthyl, 2-naphthyl, 1-anthryl, 2-anthryl, and 9-anthryl. The aryl group is more preferably a phenyl group having a substituent, and still more preferably a residue derived from an amino acid having a phenyl group or a residue derived from a peptide having a phenyl group.

Here, the "residue derived from an amino acid having a phenyl group" means a group obtained by removing one hydrogen atom on a phenyl group from an amino acid having a phenyl group (for example, tyrosine or phenylalanine optionally substituted with a halogen atom or the like).

Examples of the "substituent" in the aryl group include a group capable of specifically binding to a target molecule. Here, examples of the target molecule include an antigen, a transporter, a receptor, an enzyme, a gene, and the like specifically or excessively expressed in a cancer cell. Examples of such a "substituent" include a C₁₋₆ alkyl group substituted with a carboxy group and an amino group (preferably methyl or ethyl); a carboxy group; an amino group; a guanidino group; a group having a tropane skeleton; a fatty acid residue (a group obtained by removing any one hydrogen atom from a fatty acid); and a residue of biological substances such as peptides, proteins, antibodies, or nucleic acids (a group obtained by removing any one hydrogen atom from biological substances).

The compound S can be preferably an amino acid derivative, a peptide, or a protein.

Examples of the amino acid derivative include an aromatic amino acid such as tyrosine, phenylalanine, tryptophan, or histidine, and a derivative thereof. Examples of the derivative of an aromatic amino acid include a compound in which an amino group of an aromatic amino acid is substituted with an acetamide group. Preferably, the compound S is a tyrosine derivative. In addition, the compound S can also be a compound having a phenol structure, an indole structure, or an aniline structure.

The type of the protein is not particularly limited, and is preferably an antibody or a fragment of an antibody, and particularly preferably an antibody or a fragment of an antibody that specifically binds to a protein associated with a disease. By binding a radionuclide to such an antibody or a fragment of an antibody, the radionuclide can be delivered to a site that causes a disease (for example, tumor tissue or the like), and the radiation emitted by the radionuclide can kill cells and tissues that cause the disease. Examples of the antibody that specifically binds to a protein associated with a disease include rituximab, which is an anti-CD20 antibody, and trastuzumab, which is an anti-HER2 antibody. As the radionuclide to be bound to such an antibody, ²¹¹At that emits α-rays, ¹³¹I that emits β-rays, and the like are preferable. The antibody fragment may be any fragment as long as the binding property of the antibody is not lost. Specific examples of the antibody fragment include a Fab fragment, an F(ab')₂ fragment, and a single-chain variable fragment (scFv).

The peptide is also not particularly limited, and it is preferable to use a substance associated with a disease (a protein, a sugar chain, or the like) or a peptide exhibiting affinity for cells.

A concentration of the compound S in the solution B is not particularly limited, and is preferably 0.00001 M to 0.5 M, and more preferably 0.0001 M to 0.2 M, with respect to 1 Bq to 1,000 GBq of the radionuclide, from the viewpoint of reaction efficiency and economic efficiency.

When the concentration is low, the reaction efficiency decreases, and when the concentration is high, isolation and purification become difficult, such that an appropriate concentration range is preferable.

In a preferred aspect, the compound S is a compound represented by the following Formula (I) or a salt thereof: 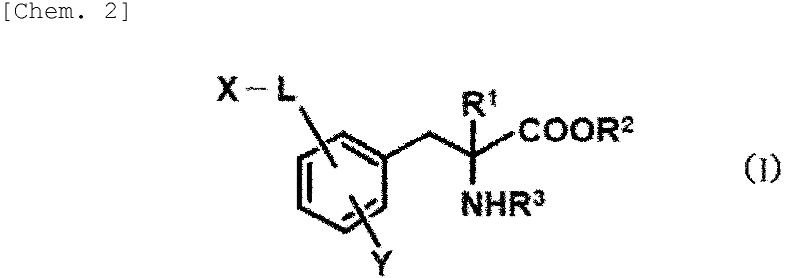 in Formula (I), X is a halogen atom; Y is a hydrogen atom or 1 to 4 arbitrary substituents; L is a direct bond or an optionally substituted C₁-C₅ alkylene group; R¹ is a hydrogen atom or an optionally substituted C₁-C₅ alkyl group; R² is a hydrogen atom, a C₁-C₅ alkyl group, a C₆-C₁₄ aryl group, or a C₇-C₁₆ arylalkyl group; and R³ is a hydrogen atom, a C₁-C₃ alkyl group, or an acyl group.

Preferably, at least one Y is a hydroxyl group. A representative example in this case is a compound having a tyrosine skeleton represented by the following Formula (II) or a salt thereof.

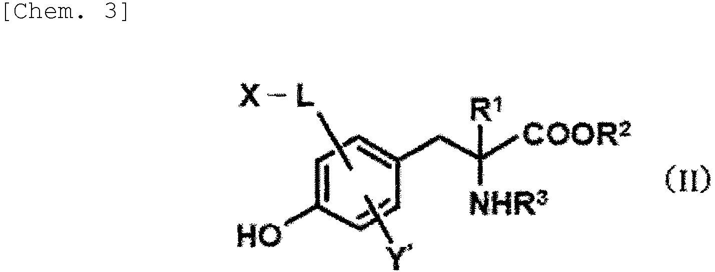

In Formula (II), X, L, R¹, R², and R³ are as defined in Formula (I), and Y' is a hydrogen atom or 1 to 4 arbitrary substituents.

In Formulas (I) and (II), R³ is preferably an acyl group, and more preferably an acetyl group.

As a representative example of Formulas (I) and (II), the compound S is N-acetyl-3-iodo-L-tyrosine.

A temperature of each step in the production method of the present invention is not particularly limited, and is preferably 5 to 50°C, and more preferably 20 to 40°C.

In a preferred aspect, each step of the present invention can be performed while allowing the solution A and/or the solution B to flow through a flow path at an arbitrary flow rate. Preferably, the application of the voltage in the step a) is performed in an electrolytic bath installed in a flow path. In this case, it is preferable to use a flow electrolysis apparatus described below. When the step is performed while flowing the solution in the flow path, unlike an electrolytic oxidation reaction that does not allow the solution to flow, an active species predicted to be AtOI²⁻, AtOICl-, AtO(OH)I-, or the like can be first produced and then continuously act on the compound S, such that side reactions such as excessive oxidation and halogenation can be suppressed. The appropriate pH, concentration, voltage, and the like in each step are organically related to realize the reaction in the flow path.

In the production method of the present invention, it is possible to obtain a radiolabeled aryl compound with a high radiochemical yield of preferably 50% or more, more preferably 55.9% or more, and still more preferably 56.3% or more. In addition, the radiolabeled aryl compound can have a radiochemical purity of preferably 71.5% or more, more preferably 80.9% or more, and still more preferably 85% or more. In a preferred aspect, according to the production method of the present invention, it is possible to achieve both two values of a radiochemical purity of 71.5% or more and a radiochemical yield of 55.9% or more.

The obtained radiolabeled aryl compound can be purified according to a conventional method using chromatography or the like, and when the solutions A and B are aqueous solutions, the aryl compound can be immediately formulated into an injection or the like without isolation.

The radiolabeled aryl compound obtained by the production method of the present invention or the compound S used in the solution B can be in the form of a salt. Such a salt is not particularly limited, and examples thereof include a base addition salt, an acid addition salt, and an amino acid salt. Examples of the base addition salt include an alkali metal salt such as a sodium salt, a potassium salt, or a lithium salt; an alkaline earth metal salt such as a calcium salt or a magnesium salt; an ammonium salt such as a tetramethylammonium salt or a tetrabutylammonium salt; and an organic amine salt such as a triethylamine salt, a methylamine salt, a dimethylamine salt, a cyclopentylamine salt, a benzylamine salt, a phenethylamine salt, a piperidine salt, a monoethanolamine salt, a diethanolamine salt, a tris(hydroxymethyl)methylamine salt, a lysine salt, an arginine salt, a N-methyl-D-glucamine salt, or a morpholine salt. Examples of the acid addition salt include a mineral salt such as a hydrochloride, a hydrobromide, sulfate, nitrate, or phosphate; and an organic acid salt such as methanesulfonic acid, benzenesulfonic acid, p-toluenesulfonic acid, acetic acid, propionate, tartaric acid, fumaric acid, maleic acid, malic acid, oxalic acid, succinic acid, citric acid, benzoic acid, mandelic acid, cinnamic acid, lactic acid, glycolic acid, glucuronic acid, ascorbic acid, nicotinic acid, or salicylic acid. Examples of the amino acid salt include a glycine salt, an aspartic acid salt, and a glutamic acid salt. In addition, the salt may be a metal salt such as an aluminum salt.

In addition, when the radiolabeled aryl compound or the compound S is an amino acid or a derivative thereof, the radiolabeled aryl compound or the compound S can be D-form or L-form, but the substrate of the transporter is preferably L-form. In addition, these compounds may have one or two or more asymmetric carbon atoms depending on the type of substituent, and a stereoisomer such as an optical isomer or a diastereoisomer may be present. A stereoisomer in pure form, any mixture of stereoisomers, a racemate, and the like are all encompassed within the scope of the present invention.

The radiolabeled aryl compound or the compound S or the salt thereof may also be present as a hydrate or a solvate, and all of these substances are included within the scope of the present invention. The type of solvent for forming the solvate is not particularly limited, and examples thereof include solvents such as ethanol, acetone, and isopropanol.

### 3. Flow Electrolysis Apparatus

In another aspect, the present invention also relates to a flow electrolysis apparatus that is suitably used in the production method described above. The flow electrolysis apparatus has the following configuration:
a plurality of inlets for injecting a solution;
a plurality of flow paths through which the solution flows, the plurality of flow paths extending from the inlets;
a mixing point at which the plurality of flow paths merge;
a discharge flow path extending downstream from the mixing point;
an outlet connected to the discharge flow path;
an electrolytic bath provided between any one of the plurality of inlets and the mixing point;
a voltage application device connected to the electrolytic bath; and
a liquid feeding pump for feeding the solution at a desired flow rate.

The flow electrolysis apparatus according to the present invention preferably further includes a sample injection portion for adding a solution containing a radionuclide between the inlet and the electrolytic bath.

More specifically, the flow electrolysis apparatus of the present invention has the configuration illustrated in Fig. 1 used in Example 1 described below. Here, as each component such as the flow path or the liquid feeding pump, a member known in the art can be used. The electrolytic bath and the voltage application device are as described above. A flow rate of the solution in the flow path can be, for example, in a range of 0.01 to 0.2 mL/min.

By using such a flow electrolysis apparatus, it is possible to perform an electrolytic oxidation reaction while allowing a reaction solution to flow without limited to the case of performing an electrolytic oxidation reaction in a solution stored in a bulk reaction vessel as in the conventional electrolytic oxidation reaction, such that it is also possible to automate a reaction step.

### 5. Stopped-Flow Type Electrolysis Apparatus

Note that, in the production method, the use of the flow electrolysis apparatus has the advantage that the surface area of the electrode can be easily increased, but it is also possible to use an apparatus that does not allow an electrolyte to flow, such as a stopped-flow type electrolysis apparatus. In this case, the production method can be performed by securing the surface area of the electrode required for the electrolytic oxidation step.

### Examples

Hereinafter, the present invention will be described in more detail with reference to Examples, but the present invention is not limited to these Examples.

### [Example 1]

### 1. ²¹¹At Labeling Utilizing Electrolytic Oxidation Reaction

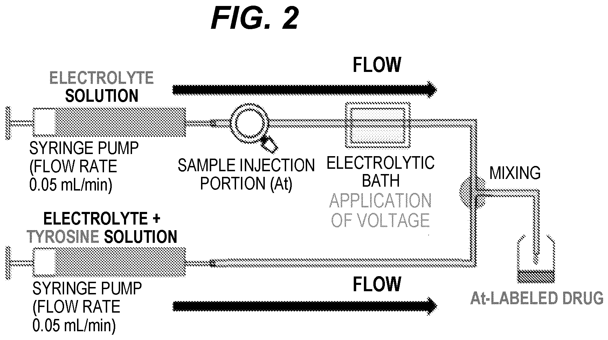

²¹¹At labeling to N-acetyl-3-iodo-L-tyrosine was performed using a flow electrolysis apparatus illustrated in Fig. 2.

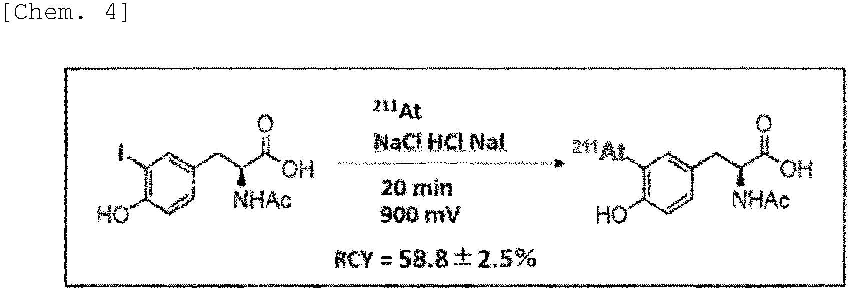

By using the apparatus of Fig. 2, a path of electrolytic oxidation of ²¹¹At and a path for feeding N-acetyl-3-iodo-L-tyrosine are different, and the electrolytically oxidized ²¹¹At is combined with N-acetyl-3-iodo-L-tyrosine in a mixed flow path. In the present apparatus, a radical reaction does not occur, and only an electrophilic substitution reaction can be observed. Specific conditions and procedures are shown below.

### <Solution Conditions>

A ²¹¹At solution was prepared under a condition of 990 mM NaCl/10mM HCl/NaI ([NaI] =100 mM or 900 mM), and used after 30 minutes or longer in order to stabilize the chemical species. As a tyrosine solution, an aqueous solution containing 10 mM N-acetyl-3-iodo-L-tyrosine and 990 mM NaCl/10 mM HCl was used. As an electrolyte solution, a 990 mM NaCl/10 mM HCl aqueous solution was used.

### <First Experimental Protocol (Flow Type)>

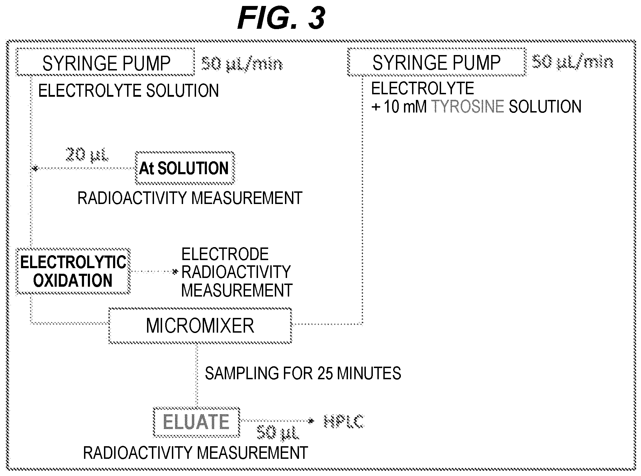

The experiment was performed according to the procedure illustrated in Fig. 3. An electrolyte solution and the tyrosine solution were flowed from two syringe pumps at a flow rate of 50 µL/min, respectively. After conditioning for 20 minutes, a voltage of 0 to 1,000 mV (vs Ag/AgCl) was applied, and 20 µL of a ²¹¹At solution was injected with an injector to perform electrolysis. An eluate was sampled for 25 minutes. 50 µL of the eluate was analyzed by HPLC. The elution behavior of the solution was examined using a UV absorption analyzer and a NaI scintillation counter as detectors, and a radiochromatogram and a UV chromatogram at that time were obtained. Analysis was performed using MeOH: 100 mM and acetate buffer = 0.50:0.25 mL/min as an eluent.

In addition, in order to examine an elution rate of ²¹¹At from the apparatus, three samples of the ²¹¹At solution (20 µL) in the same amount as the ²¹¹At solution injected into the electrolysis apparatus, the ²¹¹At solution eluted from the electrolysis apparatus, and the used electrode were collected, and radioactivity was measured with a Ge semiconductor detector. In addition, in order to examine whether the solution was eluted from the column of HPLC, the solution eluted from HPLC was sampled for 20 minutes, and radioactivity was measured with a Ge semiconductor detector.

As a result of analyzing the eluate after mixing the electrolytically oxidized ²¹¹At solution and tyrosine solution, a ²¹¹At labeled product (N-acetyl-3-astato-L-tyrosine) was obtained. A radiochemical purity was 58.8% (± 2.5%).

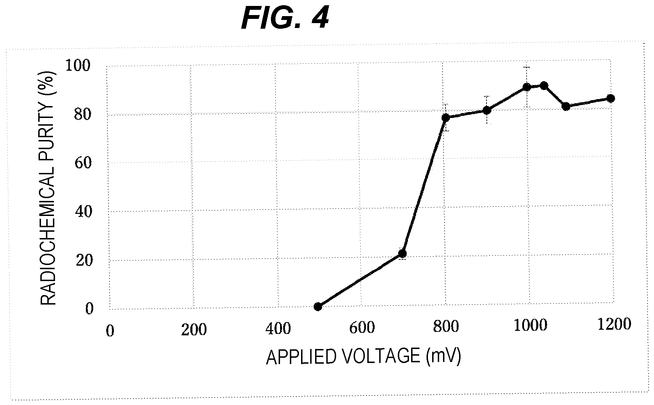

Here, the dependence of the applied voltage in the electrolytic oxidation on the yield was examined. A ratio of N-acetyl-3-astato-L-tyrosine in ²¹¹At eluted from the electrolysis apparatus (radiochemical purity of the target product in the obtained solution) is illustrated in Fig. 4 (specific numerical values are as follows).

| Applied voltage/mV | Radiochemical purity/% | Radiochemical yield/% |
|---|---|---|
| 500 mV | 0% | 0% |
| 700 mV | 21.5% | 17.5% |
| 800 mV | 77.1% | 58.1% |
| 900 mV | 79.8% | 58.8% |
| 1000 mV | 89.1% | 50.2% |
| 1040 mV | 89.6% | 10.8% |
| 1090 mV | 80.9% | 15.8% |
| 1200 mV | 84.1% | 7.19% |

As a result, it was found that application of a voltage exceeding 800 mV is preferable for ²¹¹At labeling. A purity as high as 89.1 ± 8.2% was obtained at 1,000 mV. That is, a purity of 80.9% to 97.3% was obtained. Further, at 800 mV to 900 mV, the purity of 71.5% to 85.5% was obtained. On the other hand, labeling did not proceed at 500 mV, and the value was 21% at 700 mV. It is considered that ²¹¹At oxidation chemical species produced by electrolytic oxidation causes an exchange reaction with iodine. At a voltage exceeding 800 mV, the purity tended to increase but was consistent within an error range. From this result, it was suggested that there was a boundary of a chemical species at about 800 mV, and the radiochemical purity became constant thereafter. The relationship between the applied voltage and the radiochemical purity can be described as the following mechanism. That is, when an applied voltage of about 800 mV or more is applied, the electrolytic oxidation of astatin occurs, and the chemical species is changed into a chemical species that is likely to cause a labeling reaction. Therefore, it is reasonably predicted that the electrolytic labeling reaction sufficiently proceeds at a higher applied voltage to obtain a high radiochemical purity, and even when the applied voltage is further increased, the high radiochemical purity is maintained. Therefore, from the viewpoint of purity, the applied voltage may be 800 mV or more, and there is no particular upper limit. In the existing application device, voltages of 1,200 mV, 1,400 mV, 1,600 mV, 1,800 mV, and 2,000 mV can be applied. Even when a higher voltage is applied, there is no problem in relation to purity.

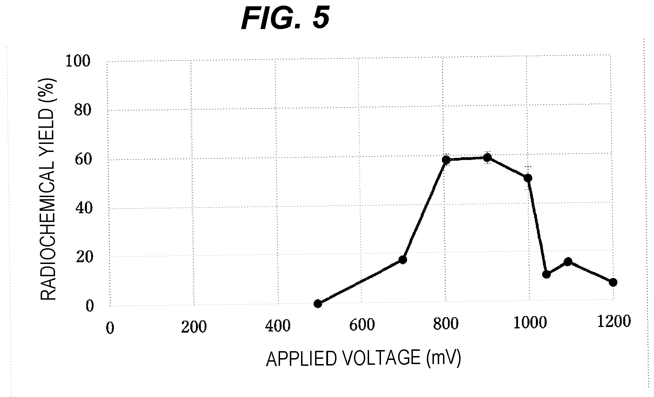

In addition, the applied voltage dependence of the radiochemical yield is illustrated in Fig. 5 (specific numerical values are as described above). As a result, the maximum yield was 900 mV, and the value was 58.8% (± 2.5%). That is, a yield of 56.3% to 61.3% was obtained. Further, at 800 mV to 900 mV, the yield of 55.9% to 61.3% was obtained. As illustrated in Fig. 3, when the applied voltage was increased, the radioactive purity was further improved. As for the yield, the yield was decreased when the applied voltage was more than 900 mV. As a result, it has been found that the radiochemical purity and the yield are well-balanced at an applied voltage in a certain range, and when the applied voltage is equal to or higher than a certain value, the radioactive purity is further improved, but the adsorption of ²¹¹At to the electrode increases with an increase in the applied voltage, and thus, the yield decreases. The relationship between the applied voltage and the radiochemical yield can be described as the following mechanism. That is, it has been found that, at an applied voltage of 800 mV or more, astatin starts to adsorb to a working electrode, such that the yield increases, but when the applied voltage exceeds 1,000 mV, most (80 to 90%) of astatin is adsorbed to the working electrode, and therefore, most of astatin is not subjected to the labeling reaction and the yield decreases when the applied voltage is 1,000 mV or more.

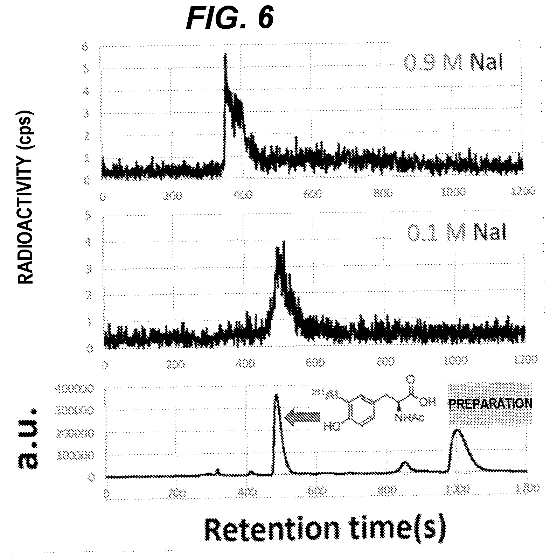

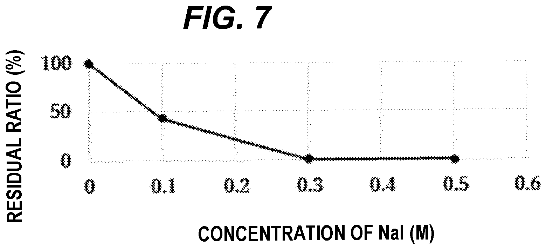

Next, a concentration of NaI added to the ²¹¹At solution was examined. This is in consideration of the possibility of production of N-acetyl-3,5-diiodo-L-tyrosine as a by-product from N-acetyl-3-iodo-L-tyrosine. Specifically, N-acetyl-3-iodo-L-tyrosine was allowed to flow through the flow path, and electrolysis was performed using NaI solutions having various concentrations. The production of N-acetyl-3-astato-L-tyrosine under each condition was identified with reference to the peak of N-acetyl-3-astato-L-tyrosine as a standard sample (preparation). (Fig. 6). At this time, a relationship between a ratio of remaining N-acetyl-3-iodo-L-tyrosine and a concentration of NaI is illustrated in Fig. 7. As a result, it was found that the concentration of NaI in the ²¹¹At solution needs to be less than 0.3 M, and preferably around 0.1 M. This is the case for the concentration of N-acetyl-3-astato-L-tyrosine in the ²¹¹At solution described in paragraph 0070, and since an allowable concentration of NaI varies depending on other conditions, it is obvious that the concentration of NaI does not always need to be less than 0.3 M.

### [Example 2]

### <Second Experimental Protocol (Stopped-Flow Type)>

Unlike the procedure illustrated in Fig. 3, the experiment was performed in a stopped flow without allowing the electrolyte to flow. Specifically, a voltage is applied to the electrolytic bath to introduce the ²¹¹At solution and the electrolyte solution into the electrolytic bath, and then the flow is stopped to perform electrolytic oxidation. After an arbitrary time, the solution is allowed to flow again and mixed with a substrate for labeling.

Even in the case of the stopped-flow type, it was confirmed whether an appropriate purity and yield could be realized under the same conditions as described above. A ratio of N-acetyl-3-astato-L-tyrosine in ²¹¹At eluted from the electrolysis apparatus (radiochemical purity of the target product in the obtained solution) was as follows.

| Applied voltage/mV | Radiochemical purity/% | Radiochemical yield/% |
|---|---|---|
| 820 mV | 67.6% | 44.7% |

The above results demonstrate that, according to the production method of the present invention, halogen in a halogenated aryl group can be substituted with a radionuclide such as ²¹¹At using an electrolytic oxidation reaction.

According to the method, not only an excellent radiochemical yield can be obtained, but also the halogenated aryl group in the starting material is stable under acid or basic conditions, such that not only the synthesis of the compound is facilitated, but also storage under low temperature conditions such as freezing becomes unnecessary. In addition, the reactivity can be easily controlled by changing the voltage applied in the electrolytic oxidation reaction, and the reaction step can be automated using the flow electrolysis apparatus. The present invention is applicable to radiolabeling of various known cancer integrated molecules such as AMT, PSMA, and FAPI.

## Claims

1. A production method of an aryl compound labeled with a radionuclide, the method comprising:
a step of applying a voltage to a solution A containing a radionuclide and electrolytically oxidizing the radionuclide; and
a step of mixing the solution A and a solution B containing a compound S having a halogenated aryl group to substitute a halogen atom on the aryl group of the compound S with the radionuclide.

2. The production method according to claim 1, wherein each of the steps is performed while allowing the solution A and/or the solution B to flow in a flow path at an arbitrary flow rate.

3. The production method according to claim 1, wherein each of the steps is performed without allowing the solution A and/or the solution B to flow.

4. The production method according to claim 2 or 3, wherein the application of the voltage is performed in an electrolytic bath installed in a flow path.

5. The production method according to any one of claims 1 to 4, wherein a radiochemical purity of the aryl compound labeled with the radionuclide is 80.9% or more.

6. The production method according to any one of claims 1 to 4, wherein a radiochemical yield of the aryl compound labeled with the radionuclide is 56.3% or more.

7. The production method according to any one of claims 1 to 4, wherein a radiochemical purity and a radiochemical yield of the aryl compound labeled with the radionuclide are 71.5% or more and 55.9% or more, respectively.

8. The production method according to any one of claims 1 to 7, wherein the voltage is 800 mV or more (vs Ag/AgCl).

9. The production method according to any one of claims 1 to 7, wherein the voltage is 800 to 1,200 mV (vs Ag/AgCl).

10. The production method according to any one of claims 1 to 7, wherein the voltage is 800 to 1,000 mV (vs Ag/AgCl).

11. The production method according to any one of claims 1 to 10, wherein the solution A contains at least one anion selected from the group consisting of an iodide ion, a bromide ion, a chloride ion, and a fluoride ion.

12. The production method according to claim 11, wherein a concentration of the anion is in a range of 0.01 M to 0.3 M.

13. The production method according to any one of claims 1 to 12, wherein the radionuclide is selected from the group consisting of ²¹¹At, ²¹⁰At, ¹²³I, ¹²⁴I, ¹²⁵I, ¹³¹I, ⁷⁶Br, and ¹⁸F.

14. The production method according to any one of claims 1 to 13, wherein the radionuclide is ²¹¹At, and the halogen is an iodine atom.

15. The production method according to any one of claims 1 to 14, wherein the aryl group in the compound S is a C₆₋₁₄ aryl group optionally having a substituent.

16. The production method according to any one of claims 1 to 15, wherein the compound S is an amino acid derivative, a peptide, or a protein.

17. The production method according to any one of claims 1 to 16, wherein the compound S is a compound represented by the following Formula (I) or a salt thereof: 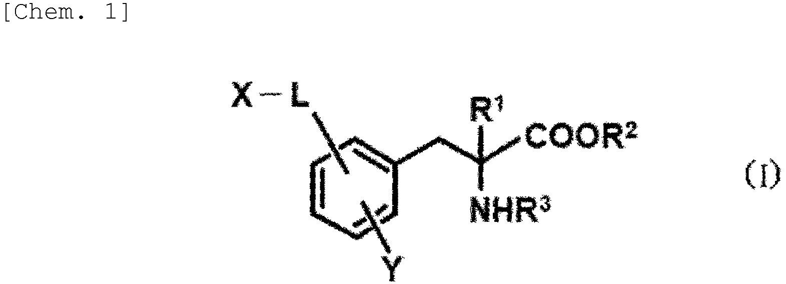 (wherein, X is a halogen atom; Y is a hydrogen atom or 1 to 4 arbitrary substituents; L is a direct bond or an optionally substituted C₁-C₅ alkylene group; R¹ is a hydrogen atom or an optionally substituted C₁-C₅ alkyl group; R² is a hydrogen atom, a C₁-C₅ alkyl group, a C₆-C₁₄ aryl group, or a C₇-C₁₆ arylalkyl group; and R³ is a hydrogen atom, a C₁-C₃ alkyl group, or an acyl group).

18. The production method according to claim 17, wherein at least one Y is a hydroxyl group and R³ is an acyl group.

19. The production method according to claim 18, wherein the protein is an antibody or an antibody fragment.

20. A flow electrolysis apparatus for use in the production method according to any one of claims 1 to 19, the flow electrolysis apparatus comprising:
a plurality of inlets for injecting a solution;
a plurality of flow paths through which the solution flows, the plurality of flow paths extending from the inlets;
a mixing point at which the plurality of flow paths merge;
a discharge flow path extending downstream from the mixing point;
an outlet connected to the discharge flow path;
an electrolytic bath provided between any one inlet of the plurality of inlets and the mixing point;
a voltage application device connected to the electrolytic bath; and
a liquid feeding pump for feeding the solution at a desired flow rate.

21. The flow electrolysis apparatus according to claim 20, further comprising a sample injection portion for adding a solution containing a radionuclide between the inlet and the electrolytic bath.

22. A stopped-flow type electrolysis apparatus for use in the production method according to any one of claims 1 to 19.

23. The stopped-flow type electrolysis apparatus according to claim 22, further comprising a sample injection portion for adding a solution containing a radionuclide.
